(19) Europäisches Patentamt
European Patent Office
Office européen des brevets

(11) **EP 1 409 637 B1**

(12) **EUROPEAN PATENT SPECIFICATION**

(45) Date of publication and mention
of the grant of the patent:
**12.03.2008 Bulletin 2008/11**

(51) Int Cl.:
*C12M 1/34* (2006.01)　　*G01N 33/487* (2006.01)
*G01N 22/00* (2006.01)　　*C12Q 1/02* (2006.01)
*G01N 21/64* (2006.01)　　*C12Q 1/04* (2006.01)
*G01N 21/62* (2006.01)　　*G01N 21/76* (2006.01)
*G01N 21/82* (2006.01)

(21) Application number: **02749038.2**

(22) Date of filing: **19.07.2002**

(86) International application number:
**PCT/GB2002/003330**

(87) International publication number:
**WO 2003/008532 (30.01.2003 Gazette 2003/05)**

(54) **APPARATUS AND METHOD FOR CONTINUOUS CELL CULTURE**

VORRICHTUNG UND VERFAHREN FÜR KONTINUIERLICHE ZELLKULTUR

APPAREIL ET PROCEDE DE CULTURE CELLULAIRE CONTINUE

(84) Designated Contracting States:
**AT BE BG CH CY CZ DE DK EE ES FI FR GB GR
IE IT LI LU MC NL PT SE SK TR**

(30) Priority: **19.07.2001 GB 0117715**

(43) Date of publication of application:
**21.04.2004 Bulletin 2004/17**

(60) Divisional application:
**07022835.8 / 1 892 287**

(73) Proprietor: **University College Cardiff Consultants
Limited
Cardiff CF24 0DE (GB)**

(72) Inventors:
• **ELLISON, Brian, Norman
Wantage,
Oxon OX12 9JP (GB)**
• **GIBSON, Colin
Cardiff CF14 6HE (GB)**
• **GRANT, Norman, Arthur
Waterlooville PO7 7NQ (GB)**
• **HYLAND, Gerard, Joseph
Southam,
Warwickshire CV47 1FQ (GB)**
• **LLOYD, David, B.
Cardiff CF15 8BR (GB)**
• **MAGEE, John, Thomas
Treharris CF46 5HQ (GB)**
• **POOLEY, David, Tallis
Hayle,
Cornwall TR27 5JB (GB)**
• **STEWART, William, Ralph, Craig
Cardiff CF5 2EN (GB)**

(74) Representative: **Newell, William Joseph et al
Wynne-Jones, Laine & James LLP
Morgan Arcade Chambers
33 St Mary Street
Cardiff CF10 1AF (GB)**

(56) References cited:
EP-A- 0 757 247　　　US-A- 4 337 648
US-A- 4 678 326　　　US-A- 4 948 975
US-A- 5 028 541　　　US-A- 5 089 385
US-A- 6 122 042

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

## Description

This invention relates to analysis apparatus and methods. The effects analysed can include cell growth and replication, the absorption and reflection of the incident radiation and also emission or other properties observed in particular wavebands as a result of irradiation of the sample, for example luminescence or fluorescence.

Scientific understanding of biochemistry has changed considerably in recent decades. Biological molecules, once perceived as rigid structures, are now known to show rapid, continuous changes in shape that are important in their biological functions.

It is known that biological effects can occur in cultures of both bacteria and yeast exposed to low-intensity microwave radiation. However, the above observations tended not to be predictable and the mechanisms involved are not yet fully understood.

It is known that the shape of a molecule is inextricably linked to its chemistry and, in a polar system, oscillatory modes correspond to frequencies of absorption of electromagnetic radiation in the microwave to infrared region. The selective deposition of electromagnetic energy modifies the population of selected modes, so changing the shape, and hence, the chemical characteristics of the molecule. Therefore microwave radiation may be used selectively to manipulate and interrogate biochemical processes remotely and non-thermally.

However, existing analytical apparatus does not make use of microwave radiation in this way due to problems in delivering microwave energy to the analytical sample and in extracting sufficient and relevant information from the sample. Furthermore, in existing observations, the electromagnetic radiation has been applied to a static culture in, e.g. a petri dish and so, at best, this analyses growth, replication etc. of the culture on a batch basis. We have predicted that there will be substantial advantages in being able to analyse a biochemical sample on a continuous basis so that measurements can be taken during all phases of cell growth.

Closed systems that minimize uncontrolled power losses have also been employed using simple constructions based on waveguide sections or using resonant cavities (see Furia, L., D. W. Hill, and O. P. Gandhi. 1986. Effect of millimeter-wave irradiation on growth of Saccharomyces cerevisiae. IEEE Trans.Biomed.Eng 33: 993-999). Although it is possible to measure the bulk dissipation of power within such a system using return and transmission losses with high accuracy, a clear understanding of the distribution and uniformity of energy deposited in the sample remains a significant challenge, particularly as the penetration depth of mm-wave radiation in lossy materials such as distilled water. Other biological effects and the property of electromagnetic radiation that can affect them include: cell genotype (affected by the power of electromagnetic radiation); growth stage (affected by the frequency of electromagnetic radiation);

cell synchrony (affected by the polarity of electromagnetic radiation); cell density (affected by the Static /extra low frequency magnetic field of electromagnetic radiation); oxygenation (affected by the duration of exposure to electromagnetic radiation); latency (affected by the modulation of electromagnetic radiation).

Fröhlich (Int. J. Quantum Chem. Vol.2, p.641 1968) postulated the existence of "microwave bio-photons". Briefly, it was proposed that all biological systems emit electromagnetic radiation, the spectral characteristics of which reveal their status and function at that instant. Similarly, the status and function of any biological system may be manipulated by means of exposure to electromagnetic radiation of given spectral characteristics. Embodiments of the present invention may be used to investigate this theory.

US-A-5 028 541 discloses a flow-through cell in which the cells are cultivated in the presence of an electromagnetic field created by a microwave generator with the purpose to better control the temperature of the culture. US-A-5 089 385 describes a method of using the flow-through cell for cultivation. US-A-4 948 975 discloses an imaging system for quantitating low-level chemoluminescent reactions in an electromagnetic field in near real-time. US-A-6 122 042 shows photometric analysis apparatus for identifying object characteristics using an array of three or more LEDs/photodetectors that detect optical characteristics in different points of a sample. US-A-4 678 326 dislcoses apparatus for simultaneous detection of luminescence and turbidity including collimating lenses disposed adjacent a cuvette.

In addition, the inventors have deduced that, in preferred techniques, it is possible to reduce frequency-dependent effects so that the absorption across a particular modulated or scanned waveband is reasonably uniform rather than exhibiting strongly frequency-dependent effects.

According to the present invention there is provided apparatus for producing a continuous culture sample as defined in Claim 1 hereinbelow.

The controller can be configured to control the first flow rate so that it corresponds with the growth rate of the culture in the container as the luminescence and turbidity signals can indicate the amount of, e.g. bacteria, present. The second flow rate will usually be fixed at rate expected to be always lower than the first flow rate. Thus, the apparatus can provide a culture sample with substantially constant properties. The controller may control the first flow rate using a Proportional Integral Derivative (PID) controller. The container may include a stirring device and/or an air outlet.

The apparatus may further include a second container to which the transfer device transfers the culture, the culture being mixed in the second container with another substance. The substance may be a buffer solution, a toxicant, fresh culture media or another agent.

The device for measuring the luminescence may include a photodetector. The device for measuring the turbidity may include a light source and a photodetector, the photodetector being arranged such that it measures light passing through the culture. The light source may be switched on and off at preset intervals, for example, the light source may be an LED set to a 50% duty cycle. The intensity of the light source may be substantially equal to the luminescence of the culture. The luminescence and tubidity signals may be output as a composite signal and decoded by the controller. Where the culture is transferred to the second container, the apparatus may measure the tubidity of the culture in the second and/or first container.

The supply device and/or the transfer device may include a pump.

The apparatus may be housed in a light tight compartment. The apparatus may further include a device for controlling the temperature in the compartment. The apparatus can further include electromagnetic screening for the compartment.

According to another aspect of the present invention there is provided a method of producing a continuous luminescent culture sample including steps of:

supplying at a first flow rate a culture medium to a container for growing;
producing a luminescence signal representing a measurement of the luminescence of the culture in the container;
producing a turbidity signal representing a measurement of the turbidity of the culture in the container;
transferring the culture from the container at a second flow rate, wherein the first flow rate is controlled in accordance with the luminescence and turbidity signals.

Whilst the invention has been described above, it extends to any inventive combination of the features above or in the following description. The invention may be performed in various ways, and an embodiment thereof will now be described by way of example only, reference being made to the accompanying drawings, in which:-

Figure 1 is a view of a test apparatus for an exposure system that is shown for the purposes of leading up to the continuous culture sample production of the present invention;
Figure 2 is a graph of measured and simulated $S_{11}$ and $S_{21}$ values in the apparatus of Figure 1;
Figure 3 is a graph of measured and simulated specific absorption rates (SAR) values for the apparatus of Figure 1;
Figure 4 is a schematic view of a microwave biochemical analyser;
Figure 5 is a schematic view of another embodiment of the analyser specialised for measuring luminescence of the sample, the apparatus including sample preparation components and assay components;

Figure 6 is a schematic view of some of the sample preparation components, and
Figure 7 is a schematic view of one of the assay components.

Initially we describe a preliminary study in relation to an exposure system for minimising artefacts such as impedance mismatch, convection effects and hotspots, and we then describe a first embodiment of microwave biochemical analyser.

In the preliminary study, the exposure system was modelled to optimise test sample response to a microwave source swept in the frequency domain. To validate the model, an irradiation cell was constructed and measurements made with an automatic network analyser.

Ansoft HFSS (available from Ansoft Corporation), a 3D solver using the finite element method was used for all simulation work. Preliminary modelling and validation were undertaken in an exposure cell that could be resolved as a simple multi-port device. Initial design concentrated on optimal dosimetry rather than the convenience of readily available culture flasks and dishes in the microbiology laboratory. A number of designs were evaluated and a two-port device, essentially a waveguide straight with the sample and holder (cuvette) inserted through the waveguide cavity, was found to be the most satisfactory. The cuvette insertion slots were positioned in the centre of the waveguide's broadside wall in order to minimise propagation into free space. In this two-port scheme, microwave radiation can be either:- i) absorbed into the cuvette and sample, ii) reflected ($S_{11}$), iii) transmitted ($S_{21}$), or iv) radiated into free space, through evanescent mode propagation or through leakage from the waveguide slot. Simulated electric field strengths in the sample can be used to derive local SAR (specific absorption rate) distribution and port "S" parameters. A quantitative evaluation for "hot spots", and regions likely to produce convection effects, was performed. Local SAR values were exported from HFSS post-processor on a Cartesian grid, with user definable spacing.

$$SAR_{local} = \frac{\sigma |E|^2}{\rho_m}$$

$\rho^m$ *mass density*

$\sigma$ *effective conductivity*

$E$ *electric field V/M*

The positioning and construction of the sample holder (cuvette) are important to optimisation of the irradiation cell. Materials selected offered a combination of biocompatibility and good microwave transmission characteristics, for example PTFE, quartz and silicon rubber. Another important parameter is sample thickness as microwave penetration is relatively superficial. Ultra-thin films provided best local SAR homogeneity but this had to be weighed against the practicalities of operating a flow system - a 0.5 mm sample bore was selected as a compromise. Tubular cuvette geometry improved local SAR homogeneity as "edge" effects were removed. A cuvette with internal diameter of 1 mm was sufficient for sample absorption of a substantial fraction of the incident power, but still gave sufficient transmission to allow determination of the cuvette's frequency-dependent absorption characteristics. Also important is impedance matching, which could be improved by selection of a low (<20 degree) insertion angle to the horizontal, although this lowers SAR. The thickness of the waveguide wall was increased to ensure that practically all radiation was absorbed and did not propagate into free space. Oxygen permeability was a further factor in selection of cuvette material.

The predictive qualities of the simulation were dependent on accurate representation of dielectric properties for reference liquids and cuvette materials. A look-up table covering the 27.5 - 35GHz region, at 25°C, was computed for both pure water and saline (3% NaCl) using the Debye equation, and a modified version with additional terms for salinity. The additional constituents of the marine culture media had little impact on dielectric properties. Values for quartz and PTFE were readily available in the literature and two values quoted for silicon were interpolated.

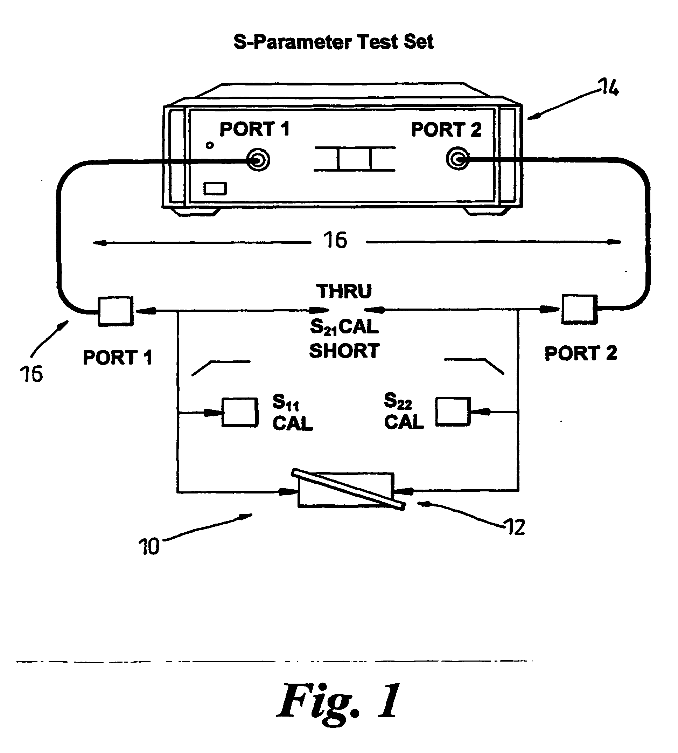

Referring to Figure 1, the simulation-optimised exposure cell 10 was fabricated from copper block and then electroplated with gold. Dicot construction allowed the sterile cuvette 12 to be located and secured by a bolting system where two symmetrical sections form the cell with the partition in the centre of the waveguide's broadside wall. The interface between the network analyser 14 and irradiation cell port was formed from a coax-waveguide adapter 16, flexible waveguide section and a waveguide bend - duplicated and positioned to form a second limb of test set-up on port two.

Acquisition of "S" parameter data was undertaken with a network analyser (8510c - Hewlett Packard) controlled remotely through an IEEE 488 interface and software written using a graphical interface language. A response calibration was performed to null both return and transmission losses in the test set-up. Measurements were made of return and transmission losses of the empty irradiation cell itself, which were negligible. Finally, the empty cuvette was inserted to calibrate out any additional losses.

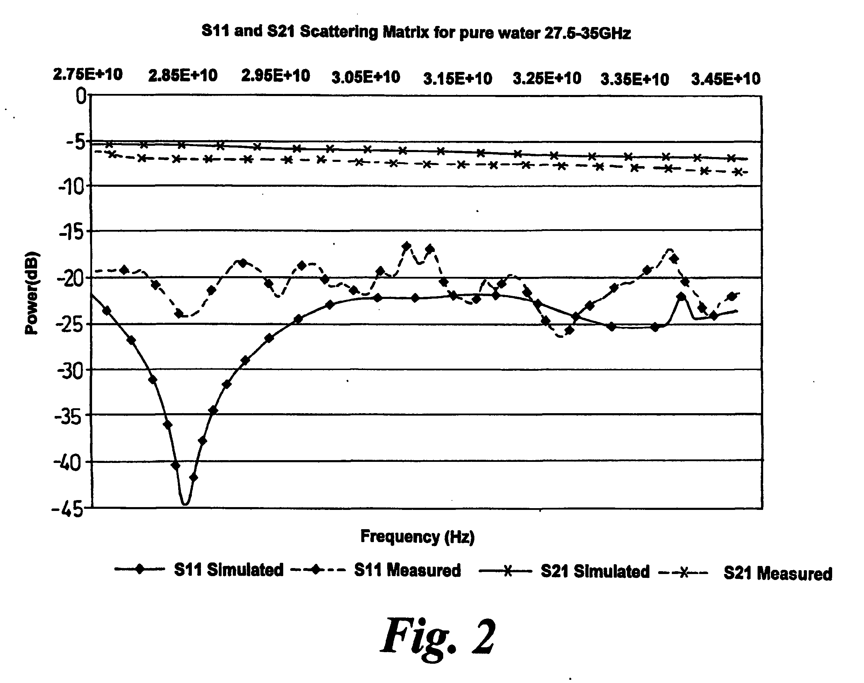

Returned ($S_{11}$) and transmitted power ($S_{21}$) were sampled at 51 points within the 27.5-35GHz frequency range. The cuvette material was PTFE with pure water as the reference sample. Temperature was maintained at 25°C +-1°C throughout the experiment. This was compared to the $S_{11}$ and $S_{21}$ parameters generated at the same frequencies, but through simulation and with identical convergence criterion for each point. Fig.2 illustrates measured and simulated $S_{11}$ and $S_{21}$ values. $S_{21}$

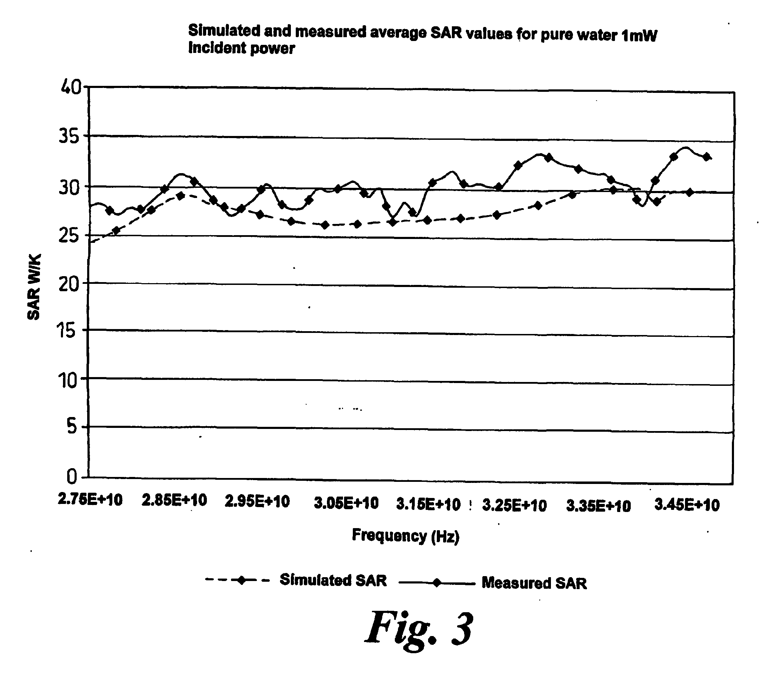

simulated and measured deviated systematically by approximately 2dB. $S_{11}$ was much smaller and although a predicted feature could not be observed experimentally, this had little impact on the total sample SAR. Fig.3 compares measured and simulated sample SAR with a source power of 1 mW, which correlated well. Variation across the band was minimised by good impedance matching.

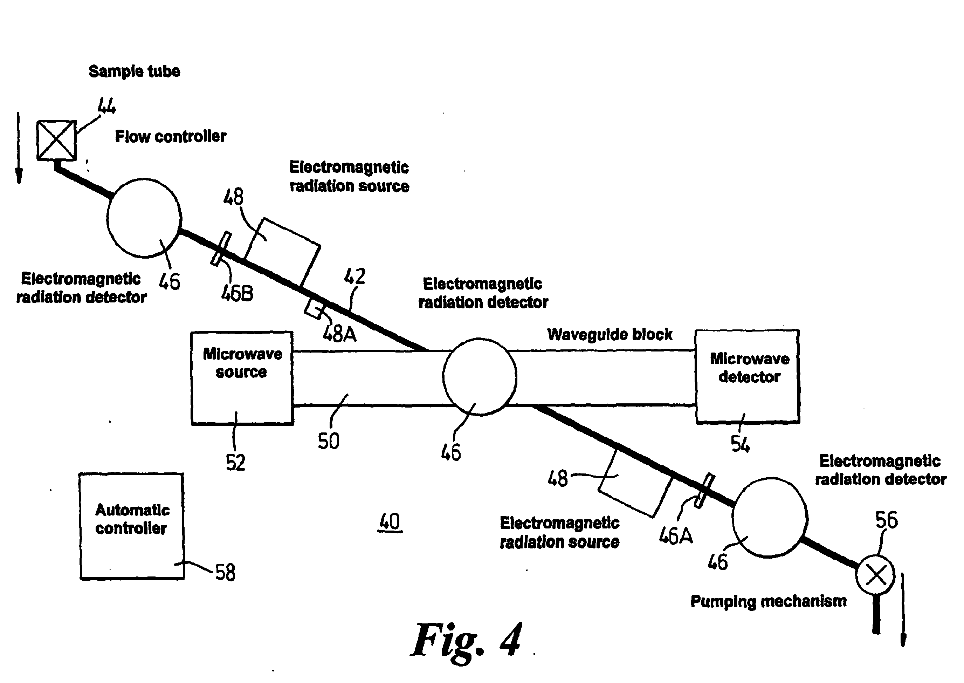

Referring now to Figure 4, the microwave biochemical analyser apparatus 40 illustrated in the drawing comprises a sample tube 42 of suitable material such as PTFE, quartz or silicone rubber having a relatively low internal diameter (typically about 1 millimetre) defines a sample path from a storage vessel (not shown) to a sample outlet (not shown) and will usually be sent to waste. The sample tube 42 defines a sample path along which various components are located. Thus the sample passes a flow controller 44 which may, for example, be a simple valve or it could be a more complex device which alters the fluid velocity profile across the cross-section of the sample tube or it may adjust the turbidity of the sample.

Three electromagnetic radiation detectors 46 are disposed at upstream, midstream and downstream positions as shown in the drawing. The radiation detectors 46 may be broad spectrum devices or they may be finely tuned to "look" for radiation in a particular defined narrow waveband. For example the detectors may be used in an I.R. Thermography process. A temperature probe or sensor 46A and a temperature control device 46B are also disposed along the sample tube 42.

Two electromagnetic radiation sources 48, which may emit radiation from a broad spectrum, e.g. microwaves, infrared light, visible light and ultraviolet light, are directed towards the sample path. Again, these may emit a broad spectrum excitation energy or this may be tuned to a particular narrow waveband. Near the centre of the sample path, the sample tube 42 passes obliquely through a waveguide block of rectangular form 50 having at one end a microwave source 52 and at the other a microwave detector 54. The sample tube 42 passes through a hole in the centre of one of the broad sides of the waveguide block and exits through a hole in the opposite wall of the waveguide block. In one embodiment an ultrasound source 48A may also be disposed along the sample tube.

A pumping mechanism 56 is disposed at the end of the sample path for drawing fluid along the path.

The various components described above are controlled by an automatic controller 58 which can perform frequency sweeping of the various radiation or energy sources or provide particular energy input profiles, and also controlling the various radiation detectors accordingly.

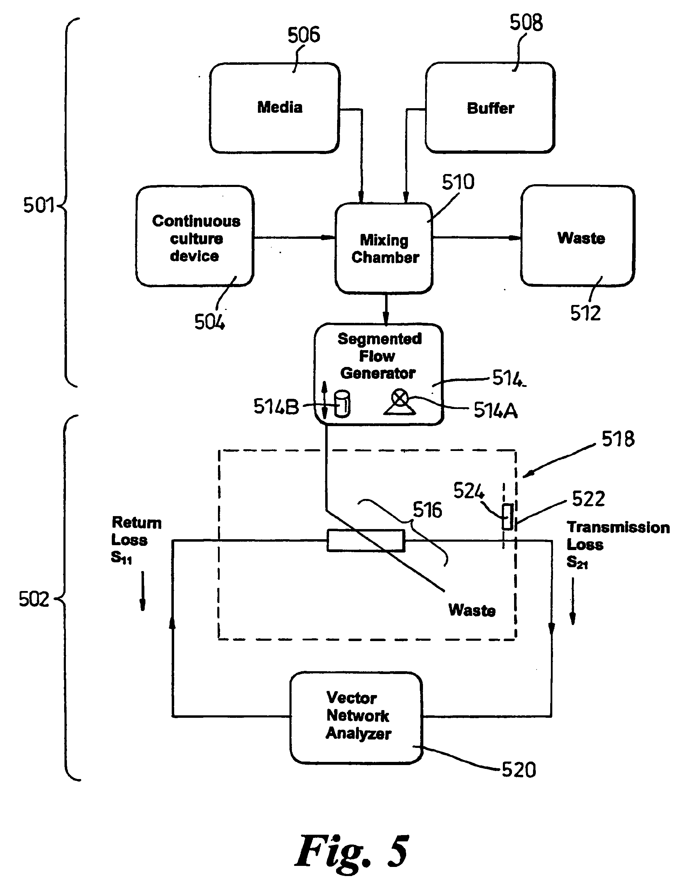

Figure 5 illustrates a further embodiment mainly intended for analysing changes in luminescence. The apparatus includes sample preparation components generally indicated at 501 for preparing a sample for analysis and assay equipment generally indicated at 502 for performing the analysis.

The sample preparation components 501 include a continuous culture production device 504, a media supply 506, a buffer supply 508, all of which are connected to a mixing chamber 510. Waste from the mixing chamber is discharged to a waste collector 512. The sample mixed in the chamber 510 is supplied to a segmented flow robot 514. The robot 514 includes a pipe 514B, one end of which is moved in and out of the sample supply in the mixing chamber 510 and pumping means 514A as described below.

The segmented sample produced by the robot 514 is supplied to the assay equipment 502, which includes an exposure cell 516 housed in an isothermal compartment 518. Data relating to the results of the exposure carried out in the cell 516 are transferred to a vector network analyser 520.

The compartment 518 is intended to exclude exogenous sources of electromagnetic radiation because Environmental variables such as static and time-varying magnetic fields, RF fields and temperature have been implicated in the induction of biological effects. No energized equipment such as pumps and motors are located in the chamber and sampled light is coupled using fiber optics to photomultiplier tubes located outside the compartment 518.

Effective electromagnetic screening is achieved by lining the exposure compartment with 2-mm mu metal sheet 522, sufficient to attenuate background field to a mean level less than 1 $\mu$T. A static D.C. field is generated within the zero-flux chamber using a Helmholtz coil set and a constant-current power supply. Field intensity is variable over the 0-120 $\mu$T range, which simulates normal physiological exposure range. The homogeneity of the magnetic field over the analysis region is better than 1%.

Bioluminescence and other biological variables are sensitive to small changes in temperature. Temperature control is achieved by circulating water through a cooling system (shown schematically at 524) including a network of copper pipe in good thermal contact with the mu-metal walls 522. The cooling system 524 also includes a water bath with integrated cooler and heater (produced by Grant, U.K.) maintains reservoir temperature as the water is circulated at a rate of 16 L min$^{-1}$. The exposure chamber is insulated. An external temperature probe using Pt100 Platinum resistance thermometry is used as the water bath thermostat, which can maintain water temperature to within $\pm$ 0.1 °C over the 5 to 50 °C range. Water bath temperature is under computer control and can be programmed to ramp or step through a given range.

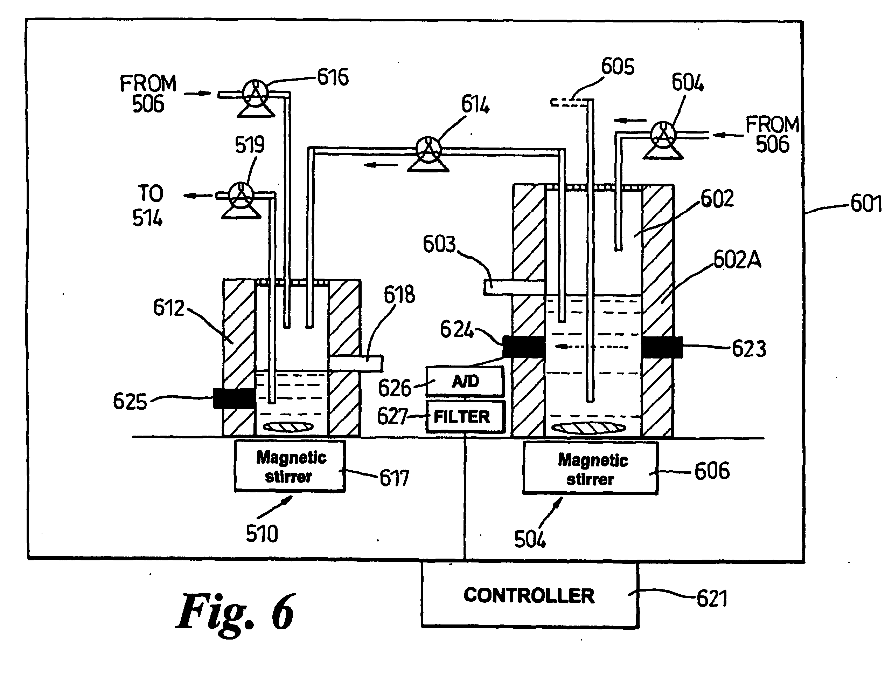

Figure 6 illustrates in more detail some of the continuous sample preparation components 501 used to supply the segmented flow robot 514.

The continuous culture production device 504 includes a fermentation vessel 602 consisting of a 50 ml "Quickfit" test tube that was modified to incorporate an overflow 603 giving a 20-ml working volume. The vessel 602 is housed within a cylindrical holder 602A. Attachments to the vessel 602, made via a three-way adapter, include a sparge tube; a drying tube 605 that acts as an air outlet and two splash-heads connected in series to prevent "grow back" into a supply reservoir (not shown) for nutrient used to feed the growing culture. The media is supplied from the store 506 for growing in the vessel 602 by means of a tube fitted with a pump 604. The media store 506 includes 10 litre autoclavable vessels, sufficient for continuous operation for many weeks.

Air can be pumped into the vessel 602 through an in-line filter (HEPA-VENT, 99.97% $\geq$0.3$\mu$m, Whatman, U.K.) at a rate of 130-ml min$^{-1}$ oxygenating the culture via a sparge tube. The drying tube 605 may be loosely packed with cotton wool to prevent contamination and maintain a small positive pressure difference between the vessel 602 and its environment. The culture vessel 602 is mounted on a small-volume magnetic stirrer 606 (Variomag mono, H+P Labortechnik, Munich, Germany) designed for continuous use and operated at 300 rev. min$^{-1}$ by means of Silicone rubber tubing connections.

The mixing chamber 510 includes a vessel 612 with a 5-ml working volume connected to the culture vessel 602 can add flexibility to the system. The mixing vessel 612 and the culture vessel 602 are connected by means of a tube fitted with a peristaltic pump 614. The pump 614 continuously transfers material from the culture vessel 602 to the mixing vessel 612 at a lower rate than that of the medium feed pump 604 that supplies the culture vessel 602 (averaged over 1 hour) so as not to deplete the culture vessel. Closed loop control is superior to an open loop although as the initiation of media flow may be intermittent, and it is not possible to directly couple the culture vessel and mixing chamber. The side arm overflow 603 maintains constant volume in the culture vessel.

The mixing vessel 612 can be configured to dilute the material transferred from the culture vessel 602 with a product pumped via a tube by a pump 616. The tube may supply a starvation buffer (as is required for oxygen measurements) from the buffer supply 508, a toxicant, or fresh media from the media store 506. The dilution rate may typically be 10-fold in the mixing vessel 602, the intention being to ensure that there is a constant amount of the medium per volume of liquid. The mixing vessel 612 also includes a side arm overflow 618 to maintain constant volume. Material discharged from the overflows 603 and 618 can pass to the waste collection component 512. The mixing vessel 612 is stirred using a stirrer 617, although due to the favourable surface area of the vessel, no additional oxygenation may be required. The sample is pumped out via a tube by a pump 519 to the segmented flow robot 514.

Alternatively, it may be desirable to introduce additional agents that may be synergistic with exposure to MW radiation or buffers that increase the sensitivity of the bioluminescence assay system. A tube/pump arrangement could be provided to supply another substance to be mixed with the material transferred from the culture vessel 602.

The culture vessel 602, stirrers 606, 617 and the mixing vessel 612 are preferably housed in a light-tight incubator 601 at 20°C $\pm$ 0.1°C. Temperature stability is crucial with medium such as Ph. phosphoreum where a 50-fold change in luminescence occurs between 20°C and 25°C.

The medium reservoir 506, peristaltic feed pumps 604, 614, 616 (101U/R produced by Watson Marlow, Cornwall, U.K.) and computer are preferably located outside the incubator 601.

The continuous culture device 504 and mixing chamber 510 are controlled by a computer-based controller 621 which actuates media feed in response to fluctuations in bioluminescence and turbidity of the sample. The computer can also be used for recording measurements relating to the luminescence of the sample being produced. The measurements may be provided by a photodetector 624 mounted in the culture vessel holder 602A. This can maximize luminous flux from the culture vessel 602, which can be considered as an area source. Turbidity can be measured optically by detecting the varying intensity of a beam of light (550 nm) produced by an LED 623 mounted in the vessel holder 602A. The LED faces the photodetector 624 and is fitted in the vessel holder 602A at a point substantially diametrically opposed to where the photodetector 624 is mounted. Thus, the photodetector 624 measures light passing through the culture vessel 602 as well as the luminescence of the material in the vessel itself.

Alternatively or additionally, a photo detector 625 may be fitted in a holder surrounding the mixing vessel 612 on a side of the vessel remote to that adjacent the culture vessel 602. Thus, the light generated by the LED 623 can pass through the culture vessel 602 as well as the mixing vessel 612 for measurement by the photo detector 625. In this case, the photo detector 624 located between the LED 623 and the photodiode 625 may be replaced by a pre-amp to aid the luminescence measurement. It will be appreciated that light source and/or light detectors may be fitted at other locations in the apparatus, depending on the type of measurement required.

The LED 625 may be driven by the controller 621 with a 50% duty cycle. As the photodetectors 624/625 receive a composite light signal when the LED is active (i.e. light produced by the culture in the vessel 602 as well as by the LED), it is necessary to decode the signals for bioluminescence and turbidity at a later stage. The LED intensity can be adjusted using a potentiometer to approximately the same value as bioluminescence. An additional adjustable gain stage can be used to condition the photodetector signal prior to digitization.

The photodetector signal can be digitized using a differential mode technique with a 12-bit (1 in 4096) A/D converter 626 (PCI-6023E, National Instruments Corp, Austin Texas) at a frequency of 1 KHz. The acquisition rate and timing are controlled by software (Labview 6.0, National Instruments) executing on the controller 621 and the incoming data is processed in a circular buffer. A digital low pass filter 628 removes noise relating to the aeration and stirring of the culture vessel. The signal is further processed to give separate channels for turbidity and luminescence at 0.5 Hz. These can be displayed in real-time and stored by the computerised controller 621. The processed signal has the requisite stability for use in the control system.

The control of the pumps 604, 614 needs to be based on a combination of measurements taken of both light emission and turbidity. In the control system reported in Wardley-Smith B, White D, and Lowe A, J.Appl.Bact 39, 337 (1975), a feed-pump was activated on reaching a preset luminescence or turbidity threshold. That culture system also included an open loop component in the form of a timer that activated the medium feed pump (in the event that it was not initiated after a preset time) by change in luminescence or turbidity. A variable "window" setting determined the decrease in the measured parameter necessary to bring about cessation of pumping. In the embodiment described with reference to Figure 6, the relative weighting of each of the control components can be selected and optimised for each organism /strain. Thus, the controller 621 can work with the complex response of culture vessel luminescence in relation to the introduction of feed medium.

Unlike "window" control systems, which pulse-modulate the feed pump, the Proportional Integral Derivative (PID) controller 621 output is proportional. The controller may control the medium feed pump 604 by means of an analogue signal. The normalised output resulting from the measurements of turbidity and luminescence and the timer were converted into an analogue signal (CIO-DDA06/JR, 12-bit D/A conversion card, Measurement Computing, Mass, U.S.A) which is supplied to the pump 604. During growth of the culture, the mean medium flow rate of the medium supply pump 604 may be about 3.7 ml h$^{-1}$, (dilution rate 0.18h$^{-1}$), with the transfer pump 614 operating at a maximum flow rate of about 3 ml h$^{-1}$. The rate of transfer by the pump 614 may be limited to 2/3 the time-averaged media feed rate by the pump 604 to prevent depletion of culture vessel volume. The introduction of fresh media into the mixing vessel 612 allows for experimentation with (but not restricted to) exponential growth phase cultures, although the software running on the controller 621 may be required to incorporate the latency between mixing vessel 612 and the assay system 502, which is variable and depends on system flow rate.

The sample preparation components 501 described above are relatively simple in construction and can be used to supply luminescent bacteria with constant properties for either laboratory use or the assay of environmental pollutants. Furthermore, bacteria can be deployed to make sensitive (< 1 nM) oxygen measurements. The culture producing device 504 may be configured, alone or in combination, as a chemostat, turbidostat or a "bioluminostat" where bacterial bioluminescence becomes the controlling variable. During experiments carried out over extended periods (e.g. over 1 week) it was found to be possible to maintain luminescence within 5% of a pre-set value, although occasionally a non-bioluminescent "mutant" became dominant; in this case light emission was irreversibly lost. The continuous culture system is also suitable for the growth of recombinant microorganisms that either constitutively express luciferase, or do so in response to stress promoter activity. The dual set point controller can have important research and industrial applications, for example, providing immediate process control or as an inferential method to optimize biomass - product yield ratios.

The continuous culture device 504 is suitable for cultivation of constitutively bioluminescent bacteria over extended periods. Its miniature design obviates some of the problems associated with running earlier devices over long time periods: on the reagent side, the bacteria utilize very small volumes of medium and on the instrumentation side, an inexpensive photodiode light detection system is time-division multiplexed, thus dispensing with the requirement for photomultiplier and high voltage power supply. The device 504 does not require additional instrumentation such as pH and dissolved oxygen sensors.

The synthesis of the luciferase system and the expression of bioluminescence in growing bacterial cultures is subject to control by many interacting factors: growth rate, oxygen concentration, N-acylhomoserine lactone autoinducers, temperature, salt and nutrient conditions and absence of catabolite repression, are some of the more clearly identified ones. As oxygen is an essential cofactor in the biochemical reactions required for bioluminescence, in an experiment where the stirrer and air supply were turned off, a sharp change in luminous intensity was found after about 4.5 min when dissolved oxygen was depleted by respiration to below the threshold at which emission is oxygen-limited. Bioluminescence then decreased rapidly, ($t_{1/2}$ = 0.34 min.), and oscillated above the "residual glow" intensity level, with a period of about 0.6 min. Restoration of stirring and air supply gave an overshoot, the "excess flash" phenomenon, which has been interpreted in terms of an accumulation of a luciferase complex under anaerobic conditions.

Although primarily intended as a generator for toxicity testing and experimental purposes, the device 504 may equally be a useful tool in the optimization of industrial processes. F Marincs, Appl.Microbiol Biotechnol 53, 536 (2000) describes the on-line monitoring of growth in batch culture using a strain of Escherichia coli engineered for constitutive bioluminescence. That paper suggests that by measuring bioluminescence an indirect measure of viability, growth and metabolic activity can be made that would otherwise require sophisticated sampling techniques such as flow cytometry. This is further supported as luciferase activity has also been shown to be proportional to biomass in growing bacterial populations of Pseudomonas fluorescens. A common problem in fermentation processes is the accumulation of a large biomass but with a sub-optimal product yield that may be obviated by on-line monitoring of bioluminescence. Furthermore, in systems where foreign genes are expressed using various promoters, further optimization may be made by measuring light emission from lux genes fused to these promoters.

The flow rates at which the apparatus operates is laminar. Laminar flow in pipes has a parabolic profile and so in the assay components of the apparatus, a detector array would have to deconvolve the signal from each detector. Due to the difficulties of deconvolving signals with other interacting physical phenomena such as diffusion and convection segmented flow is used.

An important parameter in the assay section of this instrument is the residence / transit time of the sample which, in conjunction with the mm-wave source power, determines the sample "dose". The segmented flow robot 514 includes an eight-roller micro-cassette peristaltic pump (Watson Marlow 595U) which is situated between the mixing chamber and the analysis compartment. The flow-rate is controlled via Labview software and a 16-bit D/A conversion card (PCI-DAS1602/16, Measurement Computing, Mass, U.S.A.). The peristaltic pump controls the flow as the rollers advance, compressing the tube. To minimize this action, a pump with 3 possible heads was selected and the flow was partitioned and recombined with each pulse out of phase. High compliance tubing material was also used. No Pulsing is usually detectable when the pump was operating at its lowest flow rate.

Flow segmentation can be achieved using the back-pressure generated by the eight-roller peristaltic pump and a reciprocating stainless steel (0.2 mm bore) tube that sampled the mixing tank / introduced controlled air bubbles. The reciprocating action was produced using a counter/timer board (National Instruments 6023E, USA) programmed using a Labview routine to drive a linear stepper motor. The desired length of the sample in the tube and the space / sample ratio is controlled using a software timer causing the stainless steel tube to dwell either in the culture media or in the mixer tank air space. There may be no interruption between the pipe leading from the robot 514 to the exposure cell 516.

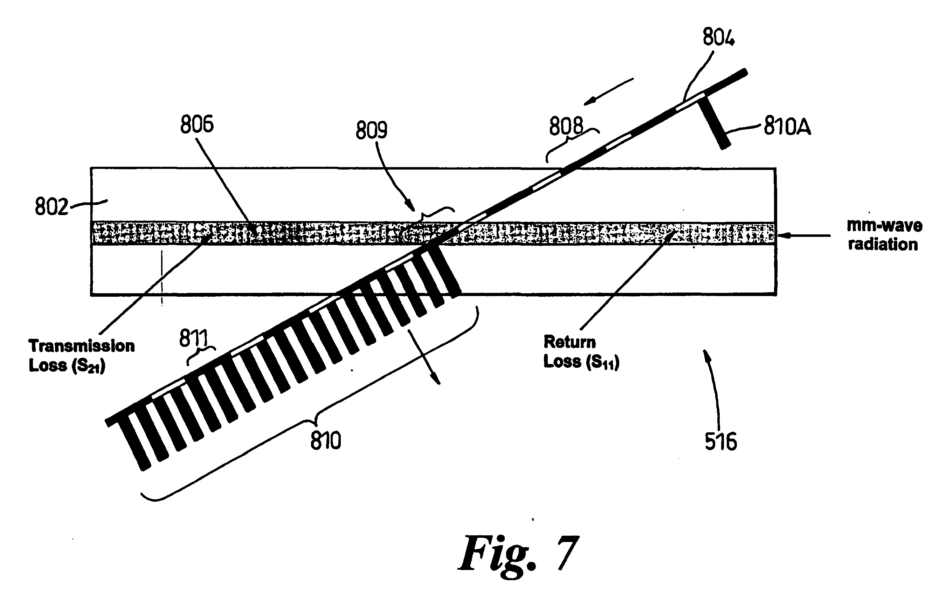

Figure 7 details the flow through exposure cell 516 contained in the isothermal compartment 518.

The flow-through exposure cell 516 is a two-port device based on a fundamental mode waveguide straight 802 with the sample tube 804 transecting the waveguide cavity 806 in the waveguide. Adjoining waveguide sections exit the exposure cell through opposing panels. High frequency electromagnetic simulation software (Ansoft, HFSS) employing the finite element method can be used to characterize exposure cell performance prior to vector network analysis by component 520. A low (< 12°) tube insertion angle improved matching characteristics across the band.

In the two-port set-up, mm-wave radiation is either i) absorbed in the sample and tube wall, ii) reflected (output at port $S_{11}$ of Figure 5), iii) transmitted (output at port $S_{21}$ of Figure 5), or propagates into free space through evanescent mode propagation (i.e. leakage) at the point where the sample tube enters the guide (unless suppressed, this propagation would represent an uncontrolled loss of signal power from the sample).

The tube insertion points, waveguide wall thickness, cuvette diameter and its material (dielectric constant) can be selected to minimize the possibility of fundamental mode waveguide propagation along the tube. The effect may be considered to be negligible, typically 30 dB lower than the power level at the centre of the cell. Tubing materials were selected on the basis of their biocompatibility, oxygen permeability and mm-wave and optical transmission characteristics.

One of the more challenging aspects of irradiation cell design relates to the microscopic deposition of power in the test sample. Inhomogeneous distribution of power can result in "hot spots" that greatly exceed the average power absorbed. Small but rapid changes in temperature can set-up convection phenomena that may incorrectly be interpreted as a non-thermal effect. Ultrathin films may provide the best spatial distribution of power within a sample. As a compromise a 0.5 mm bore may be used as this can ensure that growth on the walls of the flow system do not render it unusable too rapidly. A substantial fraction of the incident power is absorbed in this 0.5 mm sample. The rounded edges of the tube improved local SAR homogeneity as "edge" effects were removed. By simulating specific absorption within the sample, local SAR's distribution and port S parameters. A quantitative evaluation for "hot spots", and regions likely to produce convection effects, can be performed.

Biological response to mm-wave exposure is assayed using a bioluminescence-based reporter system. Light emission typically occurs in the blue-green region and is of low intensity. Due to the potentially low signal level and the desirability to improve signal to noise ratio, photon-counting photomultiplier tubes are used (H7474, Hammamatsu Corp, ). Light is sampled using a collimator, with a 2 mm aperture (Oz Optics 2522) presented to the sample tube. Collimator guides are drilled into the flow-through cell wall to monitor light during exposure. Pre and post irradiation light sampling positions are mounted along the path of the tube as it enters and exits the cell. A multimode fiber optic patchcord delivers the signal to via a SMA connector to the photomultiplier tube. Collimation means that light detector spatial resolution can be improved at the expense light source coupling detector efficiency. Each detector integrates the photon count.

The analysis system is intended to detect statistically significant changes in bioluminescence between mm-wave exposed and unexposed cell cultures as a function of parameters such as mm-wave intensity and frequency. The analysis system can either operate in a search-optimized mode using an automatic calibration system or a more statistically robust mode that incorporates both the calibration system and formal controls.

A series of collimator channels 810 are located along the portion of the sample tube 804 containing bioluminescent segments 808 that have been exposed to radiation when passing though the irradiation zone 809. As the segments 808 cross each collimator, a characteristic increase, then decrease in count rate is observed which generates a waveform that resembles the low / high states of a digital signal. A threshold algorithm can be used to detect the leading / trailing edges of each segment so that, with a known flow rate, each segment can be tracked as it passes through the detector array. Events such as step changes in frequency, power are edge triggered as new segments enter the cell 516.

The analysis is performed by integrating count rate from the central region 811 of each segment 808, which is then used to compute a statistical measure of bioluminescence that is written to a file. The central region 811 represents about 70% of the distance between the leading and trailing edges of the segment 808. Bioluminescence is measured in this way at each of the collimators in the array and compared to the pre-exposure detector value. This comparison is performed most simply by starting each detector channel sequentially, using a time delay, so that the first value in each file corresponding to each detector channel is the first segment to be analysed.

Although the delay produced by the spacing between the collimator channels is relatively short, it will be understood that the apparatus can be modified to allow the effects of exposure to radiation on the sample over a longer period of time to be investigated. For example, the sample tube may include movable valves that allow the segments to be contained for a desired period of time before being allowed to move on for measurement by the next collimator channel or before the measurement is repeated by the same channel.

The comparator system is a spreadsheet-based program that operates on the files generated by each detector channel. A ratio is calculated between intensity of bioluminescence at the pre-exposure detector and then at every other subsequent detector in the array. On the spreadsheet, this is the first column. The analysis system uses relative changes in intensity of bioluminescence. This is compared with the averaged ratios of a series of unexposed calibration segments. Sufficient segments are used in the calibration sequence to determine the basic statistics of unexposed segment bioluminescence such as standard deviation. The basic statistics of the calibration series are used to set a threshold for candidate bio-effect detection.

Unexposed calibration sequences flank exposed sequences of segments 808. A comparator program evaluates the ratio of each segment through the detector array and calculates an index of biological activity on the basis of a comparison with an unexposed series of calibration pulses.

Working in its simplest mode, the analyzer partitions segments into exposed and unexposed "calibration" segments. Calibration sequences comprise of a contiguous series of segments flanked by exposed series. The calibration series serve two purposes. First, by computing mean levels over the series, systematic drift throughout the exposure series can be fine tuned out. Secondly, the standard deviation of the calibration segment series is used to set a threshold for the detection for candidate biological effects.

It is believed that exposing the sample to radiation results in a non-thermal interaction which can change the configuration/shape of the molecular structure and the chemical properties (e.g. luminescence) of the sample. Detecting such properties can be used to provide a "fingerprint" for the sample. One example may be a healthy human tissue that may emit microwave radiation of given spectral characteristics (a function of frequency, intensity, phase, polarisation and time); however, should that tissue become pre-cancerous (or cancerous) then the spectral characteristics may change. Detection of such a change provides an opportunity for early diagnosis. Conversely, the cancerous cells may respond to irradiation with microwave radiation of certain spectral characteristics (not necessarily related to those of any emitted radiation) by initiating the death of those cells (apoptosis), while the surrounding healthy cells can remain unaffected. The apparatus may be used to experimentally determine the latter and as a research tool contributing towards establishing the existence of the former. For example, both the healthy and cancerous cells could be tagged with a luminescent (or fluorescent) protein and samples of one, then the other, could be introduced to the apparatus. The impact of various irradiation regimes could be determined by analysing the variation in light output from the respective samples.

A ratio between measured light intensity at the first pre-exposure detector 810A and the luminescence of the segments measured at each post-exposure detector station can be obtained. This part of the analysis system comprises a single "comparator" program that continuously logs data into a spreadsheet. A biological activity index is computed for each segment based on a deviation from the mean of the calibration series.

The data analysis software allows the system to operate on a very low threshold for a candidate biological effect threshold, typically twice that of the standard deviation of the calibration series. Thus, approximately 5% of the exposed segments may initially trigger as a candidate biological events. When such an event occurs an automatic repeat of that part of control parameter space is generated and the system will repeat indefinitely - thus the system can combine high sensitivity with no false positives. A feedback loop is created so that the mm-wave synthesizer delivers at increasingly higher frequency resolutions.

It should be noted that the calibration pulses effectively act as traditional control pulses in many aspects but a more formal control validation can be achieved by setting up occasional runs where a normally exposed series is left as an unexposed control.

These statistics are written to disc. The program for the collimator channel 1 (pre-irradiation waveguide) 7 also uses the segment detection, together with flow rate, to control the amplitude and frequency of the network analysis. The results can be inspected on screen for operator monitoring of the experiment if required and/or is available for interapplication operability.

For the purposes of demonstrating the apparatus' performance characteristics, the naturally bioluminescent bacterium Photobacterium phosphoreum 844 was used, which has previously been deployed in toxicity detection systems. This prototype was tested using a bacterial bioluminescence-based reporter system of the type commonly used in ecotoxicity monitoring.

The continuous culture and analytical technology described in this application differs significantly from other approaches in the respect that they are built around a continuous culture device. This supplies cells in a uniform physiological state to a flow-through exposure device for testing. Bacterial cells grown under batch conditions cease to grow exponentially when nutrient concentrations become limited. The application of continuous culture allows the biological variable to be controlled and reproducibility of experiments improved. Frequency, power density and environmental variables can be changed with respect to a test sample in a uniform physiological state. Using a flow through device it is possible to avoid problems of sequential exposure to a test sample and cumulative heating effects.

Although the total period of light emission from cultures is about 20 hours in practice, the response of luminous bacteria to toxicants may not remain constant during this period. In addition, sequential exposure to toxicants may also degrade performance of the biosensor. Therefore, where it is desirable to have a continuous supply of bacteria with constant properties, particularly constant luminescence, the continuous culture device described above may be useful.

However, the "non-substantial" nature of electromagnetic fields confer considerable advantages as one can exclude complicating factors such as absorption, distribution (in the sense of chemical barriers such as cell membranes), biotransformation and elimination. The sample in the exposure compartment is effectively maintained in stasis as the cells are supplied in a consistent physiological state, grown under defined conditions and growth rates. This configuration eliminates certain biological variables that may confound the analysis of a cell sample for sensitivity to a particular investigating parameter.

In studies designed to test the toxicity of a chemical, the term "dose" is used to describe the concentration and time to which the cells are exposed. In electromagnetic field exposure systems, "dose" is related to absorbed energy in a sample.

The system described simplifies management of power delivery to the analytical sample; it allows systematic searching in the frequency domain for biochemical effects of microwaves; it enables the monitoring of the level of a suitable reporter, for example luminescence or fluorescence, before, during and/or after microwave irradiation; it allows analytical samples to be irradiated once only, thereby avoiding cumulative effects; it facilitates the investigation of each of the relevant parameters independently from the others as required. One or more radioactive sources or generators of the same or different type can be used with one or more detectors for detecting the same or different types of radiation.

The apparatus may also include a sampling port (not shown) to enable extraction of a portion from the portion stream for physical testing independent of the system, for example plating and growth.

The apparatus may also include a device for detecting cell metabolism, cell composition, cell size, cell numbers and cell viability of a sample, either within the sample tube or extracted therefrom.

As the results of experiments that can be performed using the apparatus may be needed by persons who do not have direct access to the apparatus, a communications network can be used to transfer experiment requests and results. This can be implemented in several ways. For example, a web page may be provided that includes a form for completing details of the type of medium/media to be used, what measurements are required, the properties of the type(s) of radiation to which the medium is to be exposed, etc. These details can then be transferred over the network to a facility having the apparatus. The experiment may then be carried out in accordance with the request and the results can be transferred back to the party who made the request over the network.

**Claims**

1. Apparatus for producing a continuous culture sample including:

   a container (602) for growing a luminescent culture;
   a supply device (604) for supplying culture medium to the container; and
   a transfer device (614) for transferring the culture from the container,
   the apparatus **characterised by** being configured to produce a continuous luminescent culture sample and further including:

   a device (824) for producing a luminescence signal representing a measurement of the luminescence of the culture in the container;
   a device (624) for producing a turbidity signal representing a measurement of the turbidity of the culture in the container;
   wherein the supply device supplies the culture medium to the container at a first flow rate and the transfer device transfers the culture from the container at a second flow rate and the apparatus includes a controller (621) for controlling the first flow rate proportionally in accordance with the luminescence and turbidity signals.

2. Apparatus according to Claim 1, wherein the controller (621) controls the first flow rate using a Proportional Integral Derivative (PID) controller.

3. Apparatus according to Claim 1 or 2, wherein the container (602) includes a stirring device (606) and/or an air outlet (605).

4. Apparatus according to any one of Claims 1 to 3, further including a second container (612) to which the transfer device (614) transfers the culture, the culture being mixed in the second container with another substance.

5. Apparatus according to Claim 4, wherein the other substance includes a buffer solution, a toxicant, fresh culture media or another agent.

6. Apparatus according to any one of Claims 1 to 5, wherein the device for measuring the luminescence includes a photodetector (624).

7. Apparatus according to any one of Claims 1 to 6, wherein the device for measuring the turbidity includes a light source (623) and a photodetector (624), the photodetector being arranged such that it measures light passing through the culture.

8. Apparatus according to Claim 7, wherein the light source (623) is switched on and off at preset intervals.

9. Apparatus according to Claim 8, wherein the light source (623) includes an LED set to a 50% duty cycle.

10. Apparatus according to any one of Claims 7 to 9, wherein the intensity of the light source (623) is substantially equal to the luminescence of the culture.

11. Apparatus according to any one of Claims 7 to 10, wherein the luminescence and tubidity signals are output as a composite signal and decoded by the controller (621).

12. Apparatus according to Claim 7, wherein the photo detector (625) is arranged so that it measures the tubidity of the culture in the second container (612).

13. Apparatus according to any one of Claims 1 to 12, wherein the supply device (604) and/or the transfer device (614) includes a pump.

14. Apparatus according to any one of Claims 1 to 13, wherein the apparatus is housed in a light tight compartment (601).

15. Apparatus according to Claim 14, wherein the apparatus further includes a system (524) for controlling the temperature in the apparatus.

16. Apparatus according to Claim 14 or 15, further including electromagnetic screening (522) for the apparatus.

17. Apparatus according to any one of the preceding Claims, wherein the controller (621) is configured to control the first flow rate so that is corresponds with the growth rate of the culture in the container.

18. Apparatus according to Claim 17, wherein the second flow rate is fixed at a rate expected to be always lower than the first flow rate.

19. A method of producing a continuous luminescent culture sample, the method **characterised by** including steps of:

> supplying at a first flow rate a culture medium to a container for growing;
> producing a luminescence signal representing a measurement of the luminescence of the culture in the container;
> producing a turbidity signal representing a measurement of the turbidity of the culture in the container;
> transferring the culture from the container at a second flow rate, wherein the first flow rate is controlled proportionally in accordance with the luminescence and turbidity signals.

**Patentansprüche**

1. Vorrichtung zur Herstellung einer kontinuierlichen Kulturprobe,
mit einem Behälter (602) zum Aufziehen einer Luminiszenzkultur,
einer Zufuhreinrichtung 8604) zur Abgabe von Kulturmittel an den Behälter, und einer Übergabeeinrichtung (614) zum Übergeben der Kultur aus dem Behälter,
**dadurch gekennzeichnet, daß** die Vorrichtung so gebaut ist,
daß eine kontinuierliche luminiszente Kulturprobe erzeugt wird
und des weiteren eine Einrichtung (624) vorhanden ist, die zur Erzeugung eines Luminiszenzsignals dient, das eine Messung der Luminiszenz der Kultur in dem Behälter beinhaltet,
sowie eine Einrichtung (624) zur Erzeugung eines Trübungssignals, das eine Messung der Trübung der Kultur im Behälter beinhaltet,
wobei die Zufuhreinrichtung das Kulturmittel dem Container mit einer ersten Strömungsgeschwindigkeit zuführt und die Übertragungseinrichtung die Kultur aus dem Behälter mit einer zweiten Strömungsgeschwindigkeit überträgt und die Vorrichtung einen Regler (621) zur Regelung bzw. Steuerung der ersten Strömungsgeschwindigkeit proportional gemäß den Luminiszenz- und Trübungssignalen aufweist.

2. Vorrichtung nach Anspruch 1, **dadurch gekennzeichnet, daß** der Regler (621) die erste Strömungsgeschwindigkeit unter Verwendung eines Proportional-Integral-Differential- (PID) Reglers steuert.

3. Vorrichtung nach Anspruch 1 oder 2, **dadurch gekennzeichnet, daß** der Behälter (602) eine Rühreinrichtung (606) und / oder einen Luftauslaß (605) aufweist.

4. Vorrichtung nach einem der Ansprüche 1 bis 3, **gekennzeichnet durch** einen zweiten Behälter (612), auf den die Übertragungseinrichtung (614) die Kultur überträgt, wobei die Kultur in dem zweiten Behälter mit einer anderen Substanz vermischt wird.

5. Vorrichtung nach Anspruch 4, **dadurch gekennzeichnet, daß** die andere Substanz eine Pufferlösung, eine toxische Substanz, ein frisches Kulturmedium oder ein anderes Mittel enthält.

6. Vorrichtung nach einem der Ansprüche 1 bis 5, **dadurch gekennzeichnet, daß** die Einrichtung zur Messung der Luminiszenz einen Fotodetektor (624) aufweist.

7. Vorrichtung nach einem der Ansprüche 1 bis 6, **dadurch gekennzeichnet, daß** die Einrichtung zur Messung der Trübung eine Lichtquelle (623) und einen Fotodetektor (624) aufweist, der so angeordnet ist, daß er das Licht mißt, das durch die Kultur hindurch fällt.

8. Vorrichtung nach Anspruch 7, **dadurch gekennzeichnet, daß** die Lichtquelle (623) in vorgegebenen Intervallen ein- und ausgeschaltet wird.

9. Vorrichtung nach Anspruch 8, **dadurch gekennzeichnet, daß** die Lichtquelle (623) ein LED aufweist, das auf einen 50 % -Arbeitszyklus eingestellt ist.

10. Vorrichtung nach einem der Ansprüche 7 bis 9, **dadurch gekennzeichnet, daß** die Intensität der Lichtquelle (623) im wesentlichen gleich der Luminiszenz der Kultur ist.

11. Vorrichtung nach einem der Ansprüche 7 bis 10, **dadurch gekennzeichnet, daß** die Luminiszenz- und Trübungssignale als zusammengesetztes Signal abgegeben und durch den Regler (621) entschlüsselt werden.

12. Vorrichtung nach Anspruch 7, **dadurch gekennzeichnet, daß** der Fotodetektor (625) so angeordnet ist, daß er die Trübung der Kultur in dem zweiten Behälter (612) mißt.

13. Vorrichtung nach einem der Ansprüche 1 bis 12, **dadurch gekennzeichnet, daß** die Zufuhreinrichtung (604) und / oder die Übertragungseinrichtung (614) eine Pumpe aufweisen.

14. Vorrichtung nach einem der Ansprüche 1 bis 13, **dadurch gekennzeichnet, daß** die Vorrichtung in einer lichtdichten Kammer (601) angeordnet ist.

15. Vorrichtung nach Anspruch 14, **dadurch gekennzeichnet, daß** die Vorrichtung des weiteren ein System (524) zur Steuerung der Temperatur in der Vorrichtung aufweist.

16. Vorrichtung nach Anspruch 14 oder 15, ferner **gekennzeichnet durch** einen elektromagnetischen Schirm (522) für die Vorrichtung.

17. Vorrichtung nach einem der vorhergehenden Ansprüche, **dadurch gekennzeichnet, daß** der Regler (621) so eingestellt ist, daß er die Strömungsgeschwindigkeit so regelt, daß sie der Wachstumsgeschwindigkeit der Kultur in dem Behälter entspricht.

18. Vorrichtung nach Anspruch 17, **dadurch gekennzeichnet, daß** die zweite Strömungsgeschwindigkeit auf einen Wert eingestellt wird, von dem erwartet wird, daß er ständig unter demjenigen der ersten Strömungsgeschwindigkeit liegt.

19. Verfahren zur Erzeugung einer kontinuierlichen, luminiszenten Kulturprobe, **gekennzeichnet durch** die folgenden Schritte:

Zufuhr eines Kulturmediums mit einer ersten Strömungsgeschwindigkeit zu einem Behälter zwecks Aufzucht;
Erzeugung eines Luminiszenzsignals, das eine Messung der Luminiszenz der Kultur im Behälter beinhaltet;
Erzeugung eines Trübungssignals, das eine Messung der Trübung der Kultur in dem Behälter beinhaltet;
Übertragung der Kultur von dem Behälter mit einer zweiten Strömungsgeschwindigkeit, wobei die erste Strömungsgeschwindigkeit proportional gemäß den Luminiszenz- und Trübungssignalen gesteuert wird.

**Revendications**

1. - Appareil pour produire un échantillon de culture continue comprenant :

- un conteneur (602) pour faire croître une culture luminescente ;
- un dispositif d'alimentation (604) pour adresser un milieu de culture au conteneur ; et
- un dispositif de transfert (614) pour transférer la culture à partir du conteneur ;

l'appareil étant **caractérisé par le fait qu'**il est configuré pour produire un échantillon de culture luminescente continue, et celui-ci comprenant en outre :

- un dispositif (624) pour produire un signal de luminescence représentant une mesure de la luminescence de la culture dans le conteneur ;
- un dispositif (624) pour produire un signal de turbidité représentant une mesure de la turbidité de la culture dans le conteneur ;

le dispositif d'alimentation adressant le milieu de culture au conteneur à un premier débit et le dispositif de transfert transférant la culture à partir du conteneur à un second débit, et l'appareil comprenant un contrôleur (621) pour commander le premier débit proportionnellement conformément aux signaux de luminescence et de turbidité.

2. - Appareil selon la revendication 1, dans lequel le contrôleur (621) commande le premier débit à l'aide d'un régulateur Proportionnel, Intégral, par différentiation (PID).

3. - Appareil selon l'une des revendications 1 ou 2, dans lequel le conteneur (602) comprend un dispositif d'agitation (606) et/ou une sortie d'air (605).

4. - Appareil selon l'une quelconque des revendications 1 à 3, comprenant en outre un second conteneur (612) auquel le dispositif de transfert (614) transfère la culture, la culture étant mélangée dans le second conteneur avec une autre substance.

5.   - Appareil selon la revendication 4, dans lequel l'autre substance comprend une solution tampon, une substance toxique, des milieux de culture frais ou un autre agent.

6.   - Appareil selon l'une quelconque des revendications 1 à 5, dans lequel le dispositif pour mesurer la luminescence comprend un photodétecteur (624).

7.   - Appareil selon l'une quelconque des revendications 1 à 6, dans lequel le dispositif pour mesurer la turbidité comprend une source de lumière (623) et un photodétecteur (624), le photodétecteur étant agencé de telle sorte qu'il mesure la lumière passant à travers la culture.

8.   - Appareil selon la revendication 7, dans lequel la source de lumière (623) est allumée et éteinte à des intervalles préréglés.

9.   - Appareil selon la revendication 8, dans lequel la source de lumière (623) comprend une DEL réglée à un rapport cyclique de 50%.

10.   - Appareil selon l'une quelconque des revendications 7 à 9, dans lequel l'intensité de la source de lumière (623) est sensiblement égale à la luminescence de la culture.

11.   - Appareil selon l'une quelconque des revendications 7 à 10, dans lequel les signaux de luminescence et de turbidité sont émis en tant que signal composite et décodés par le contrôleur (621).

12.   - Appareil selon la revendication 7, dans lequel le photodétecteur (625) est agencé de telle sorte qu'il mesure la turbidité de la culture dans le second conteneur (612).

13.   - Appareil selon l'une quelconque des revendications 1 à 12, dans lequel le dispositif d'alimentation (604) et/ou le dispositif de transfert (614) comprennent une pompe.

14.   - Appareil selon l'une quelconque des revendications 1 à 13, dans lequel l'appareil est logé dans un compartiment (601) étanche à la lumière.

15.   - Appareil selon la revendication 14, dans lequel l'appareil comprend en outre un système (524) pour commander la température dans l'appareil.

16.   - Appareil selon l'une des revendications 14 ou 15, comprenant en outre un écran électromagnétique (522) pour l'appareil.

17.   - Appareil selon l'une quelconque des revendications précédentes, dans lequel le contrôleur (621) est configuré pour commander le premier débit, de telle sorte qu'il corresponde à la vitesse de croissance de la culture dans le conteneur.

18.   - Appareil selon la revendication 17, dans lequel le second débit est fixé à une quantité attendue être toujours inférieure au premier débit.

19.   - Procédé de production d'un échantillon de culture luminescente continue, le procédé étant **caractérisé par le fait qu'**il comprend les étapes consistant à :

   - adresser à un premier débit un milieu de culture à un conteneur pour amener ledit milieu à croître ;
   - produire un signal de luminescence représentant une mesure de la luminescence de la culture dans le conteneur ;
   - produire un signal de turbidité représentant une mesure de la turbidité de la culture dans le conteneur ;
   - transférer la culture à partir du conteneur à un second débit, le premier débit étant commandé proportionnellement conformément aux signaux de luminescence et de turbidité.

**S-Parameter Test Set**

*Fig. 1*

S11 and S21 Scattering Matrix for pure water 27.5-35GHz

Frequency (Hz)

S11 Simulated — S11 Measured — S21 Simulated — S21 Measured

*Fig. 2*

Simulated and measured average SAR values for pure water 1mW incident power

Fig. 3

EP 1 409 637 B1

Fig. 4

EP 1 409 637 B1

*Fig. 5*

*Fig. 6*

EP 1 409 637 B1

*Fig. 7*

EP 1 409 637 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5028541 A
- US 5089385 A
- US 4948975 A
- US 6122042 A
- US 4678326 A

### Non-patent literature cited in the description

- **FURIA, L. ; D. W. HILL ; O. P. GANDHI.** Effect of millimeter-wave irradiation on growth of Saccharomyces cerevisiae. *IEEE Trans.Biomed.Eng,* 1986, vol. 33, 993-999
- **FRÖHLICH.** *Int. J. Quantum Chem.,* 1968, vol. 2, 641
- **WARDLEY-SMITH B ; WHITE D ; LOWE A.** *J.Appl.Bact,* 1975, vol. 39, 337
- **F MARINCS.** *Appl.Microbiol Biotechnol,* 2000, vol. 53, 536